# EUROPEAN PATENT APPLICATION

(11) **EP 4 537 872 A1**
(43) Date of publication of application: **16.04.2025**
(21) Application number: 23864966.9
(22) Date of filing: 20.04.2023
(51) Int. Cl.: A61M 5/142, A61M 5/158

(54) **DRUG SOLUTION ADMINISTRATION DEVICE**

(30) Priority: 13.09.2022 JP 2022145090
(71) Applicant: Terumo Kabushiki Kaisha, Tokyo 151-0072 (JP)
(72) Inventor: YANAGISAWA, Katsuya, Ashigarakami-gun, Kanagawa 259-0151 (JP); HASEGAWA, Makoto, Ashigarakami-gun, Kanagawa 259-0151 (JP); SASAKI, Shohei, Ashigarakami-gun, Kanagawa 259-0151 (JP)
(74) Representative: KIPA AB
(86) International application number: PCT/JP2023/015710
(87) International publication number: WO 2024/057600

(57) **Abstract**

A liquid medicine administration device (10) includes a connection unit (19) that is capable of connecting a circuit (D) at a non-puncture position where a puncture needle (72) does not puncture skin (S) and disconnecting the circuit (D) at a puncture position. When the disconnection of the circuit (D) is detected as the puncture needle (72) moves to the puncture position, a controller (17) controls a drive unit (52) to move a plunger (46) of a prefilled syringe (14) toward a distal end to administer a liquid medicine (M).

## Description

### Technical Field

The present invention relates to a liquid medicine administration device that punctures skin of a living body with a puncture needle to administer a liquid medicine.

### Background Art

International Publication No. 2013/141351 discloses a liquid medicine administration device for puncturing skin of a user (patient) to be administered with a puncture needle to administer a liquid medicine. The liquid medicine administration device can automatically administer the liquid medicine by driving forces of first and second drive units. The liquid medicine administration device includes a syringe filled with the liquid medicine, a syringe needle disposed at a distal end of the syringe, and a plunger movably disposed inside the syringe. With an operation of an operator, the syringe needle moves toward the skin by the first drive unit, the skin of a living body is punctured by the syringe needle, and then, the plunger is moved toward the syringe needle by the second drive unit. The liquid medicine in the syringe is subcutaneously administered from the syringe needle by the movement of the plunger.

### Summary of Invention

In a liquid medicine administration device of International Publication No. 2013/141351, in a case where the first drive unit is not operated for some reason and the second drive unit is driven when the syringe needle does not puncture the skin, the liquid medicine is pushed out toward the puncture needle by the movement of the plunger so that the liquid medicine leaks from the puncture needle.

An object of the present invention is to solve the problem described above.

(1) An aspect of the present invention is a liquid medicine administration device including: a housing having a contact surface that has contact with skin of a living body; a needle member that includes a puncture needle and is movable relative to the housing, the puncture needle being capable of protruding from the contact surface toward the skin; a syringe that is housed in the housing and includes a cylindrical body, filled with a liquid medicine and connected to the puncture needle, a gasket movably housed in the cylindrical body, and a plunger having a distal end to which the gasket is coupled; a drive unit that is housed in the housing and moves the plunger toward the distal end by being supplied with electric power; a controller that is housed in the housing and controls the drive unit, the controller constituting a circuit with the puncture device including the needle member; and a connection unit that connects the circuit at one position of a puncture position where the puncture needle has moved toward the skin and punctures the skin and a non-puncture position where the puncture needle does not puncture the skin, and disconnects the circuit at another position of the puncture position and the non-puncture position, wherein the controller controls the drive unit to move the plunger toward the distal end when connection or disconnection of the circuit is detected as the puncture needle moves to the puncture position.

According to this liquid medicine administration device, it is possible to move the plunger toward the distal end by the drive unit after the movement of the puncture needle to the puncture position is detected by detecting the connection or the disconnection of the circuit due to the connection unit. Therefore, at the non-puncture position where the puncture needle does not puncture the skin, the liquid medicine is prevented from leaking from the puncture needle due to erroneous driving of the drive unit.

(2) In the liquid medicine administration device according to (1), the connection unit may include a first circuit component and a second circuit component, the first circuit component may be made of a metal material and disposed on the needle member, the second circuit component may be made of a metal material and disposed in the housing, the circuit may be connected when the first circuit component and the second circuit component have contact with each other, and the circuit may be disconnected when the first circuit component and the second circuit component are separated from each other.

With this configuration, a contact state and a non-contact state between the first circuit component and the second circuit component can be easily switched by moving the needle member inside the housing, and accordingly, the circuit can be connected or disconnected.

(3) In the liquid medicine administration device according to (2), the circuit may be connected when the first circuit component and the second circuit component have contact with each other at the non-puncture position, and the circuit may be disconnected when the first circuit component and the second circuit component are separated from each other at the puncture position.

With this configuration, when the puncture needle punctures the skin, the needle member moves toward the skin, and the first circuit component and the second circuit component are separated from each other to release the contact between the first circuit component and the second circuit component, whereby the circuit is disconnected. As a result, the controller can confirm the puncture position instead of the non-puncture position due to the disconnection of the circuit.

(4) In the liquid medicine administration device according to (2), the circuit may be disconnected when the first circuit component and the second circuit component are separated from each other at the non-puncture position, and the circuit may be connected when the first circuit component and the second circuit component have contact with each other at the puncture position.

With this configuration, when the puncture needle punctures the skin, the needle member moves toward the skin, and the first circuit component and the second circuit component have contact with each other, whereby the circuit is connected, and the controller can confirm the puncture position instead of the non-puncture position due to the connection of the circuit.

(5) The liquid medicine administration device according to any one of (2) to (4) may include an abutting member that has an abutting surface having contact with the skin and that is provided movably with respect to the housing, the abutting surface being capable of protruding from the contact surface, the second circuit component may be disposed on the abutting member, and the abutting member may move relative to the housing in such a manner as to maintain contact between the abutting surface and the skin when a relative position between the housing and a portion of the skin facing the abutting surface changes in a state where the housing has contact with the skin.

With this configuration, in a case where a relative position between a facing portion of the skin facing the abutting surface and the contact surface of the housing changes due to a body movement of a patient or the like in a state where the contact surface of the housing has contact with the skin, the abutting member moves relative to the housing so as to follow the facing portion of the skin. Therefore, removal of the puncture needle of the needle member from the skin is prevented, and the abutting member and the needle member do not move relative to each other, so that the contact state or the non-contact state between the first circuit component and the second circuit component is suitably maintained.

(6) In the liquid medicine administration device according to (5), the abutting member may include a protruding portion that protrudes from the contact surface of the housing when the abutting surface of the abutting member does not have contact with the skin, the protruding portion may have the abutting surface, and the abutting member may be pushed by the skin to house the protruding portion in the housing when the contact surface of the housing has contact with the skin.
(7) The liquid medicine administration device according to (5) or (6) may include a following biasing member that is provided in the housing and biases the abutting member toward the skin.

With this configuration, since an abutting state of the abutting surface of the abutting member with respect to the skin is maintained by the following biasing member, the removal of the needle member (puncture needle) is reliably prevented.

(8) The liquid medicine administration device according to any one of (1) to (7), including a puncture biasing member that is provided in the housing and biases the needle member toward the skin.

With this configuration, the contact state between the first circuit component and the second circuit component can be easily and reliably switched by the puncture biasing member for biasing the puncture needle of the needle member toward the skin. Therefore, the contact state between the first circuit component and the second circuit component can be switched without increasing the number of components of the liquid medicine administration device. The puncture biasing member can cause the puncture needle of the needle member to protrude and reliably puncture the skin.

(9) In the liquid medicine administration device according to any one of (5) to (7), a puncture mechanism that causes the needle member to protrude toward the skin from the abutting surface of the abutting member may be provided, the puncture mechanism may include a pusher that has a first inclined portion inclined with respect to a puncture direction of the puncture needle and is movable in the puncture direction with respect to the housing, a puncture biasing member that is provided in the housing and biases the needle member toward the skin, and a second inclined portion that is provided in the needle member, is inclined with respect to the puncture direction, and abuts on the first inclined portion, the connection unit may be disposed between the abutting member and the needle member, the needle member may rotate from a first position to a second position around an axis along the puncture direction, via the first inclined portion and the second inclined portion, as the pusher moves in the puncture direction, and the needle member reaching the second position may move in the puncture direction by a biasing force of the puncture biasing member.

With this configuration, the contact state between the first circuit component and the second circuit component disposed on the needle member can be switched when the needle member rotates from the first position to the second position.

(10) In the liquid medicine administration device according to (9), the abutting member may include a protruding portion protruding from the contact surface of the housing when the abutting surface of the abutting member does not have contact with the skin, the protruding portion may have the abutting surface, the abutting member may be pushed by the skin to house the protruding portion in the housing when the contact surface of the housing has contact with the skin, and the pusher may be housed in the housing when the abutting surface of the abutting member does not have contact with the skin, and protrude from the housing when the contact surface of the housing has contact with the skin.

With this configuration, since the pusher is housed in the housing when the contact surface of the housing is not in contact with the skin, so that an operator is prevented from erroneously pushing the pusher to perform erroneous puncture. In addition to the effect of the above (2), both the erroneous puncture prevention and the puncture detection are achieved.

(11) In the liquid medicine administration device according to any one of (8) to (10), the needle member may include a needle hub that holds the puncture needle, and the puncture biasing member may be disposed between the abutting member and the needle hub.

According to the present invention, the plunger can be moved toward the distal end by the drive unit after the movement of the puncture needle to the puncture position is detected by detecting the connection or disconnection of the circuit by the connection unit. Therefore, at the non-puncture position where the puncture needle does not puncture the skin, the liquid medicine is prevented from leaking from the puncture needle due to erroneous driving of the drive unit.

### Brief Description of Drawings

Fig. 1 is a partially-omitted perspective view of a liquid medicine administration device according to an embodiment of the present invention.
Fig. 2 is an enlarged plan view of the vicinity of a puncture device of the liquid medicine administration device in Fig. 1.
Fig. 3 is a cross-sectional view of the puncture device of the liquid medicine administration device illustrated in Fig. 2.
Fig. 4 is a cross-sectional view of a distal portion of a housing taken along line IV-IV in Fig. 2.
Fig. 5 is an exploded perspective view of the puncture device illustrated in Fig. 4.
Fig. 6 is a configuration diagram of a circuit in the puncture device.
Fig. 7 is a cross-sectional view illustrating a non-puncture position of the puncture device taken along line VII-VII in Fig. 3.
Fig. 8 is an external perspective view of the puncture device illustrated in Fig. 4.
Fig. 9 is an overall front view of the puncture device illustrated in Fig. 8.
Fig. 10 is an exploded perspective view in which a first circuit component is detached from a needle hub.
Fig. 11 is an exploded perspective view in which a second circuit component is detached from an abutting member.
Fig. 12 is a cross-sectional view of a state where the housing of the liquid medicine administration device illustrated in Fig. 3 is brought into contact with skin of a patient.
Fig. 13 is an external perspective view of a puncture mechanism at the time of puncture.
Fig. 14 is a cross-sectional plan view of the abutting member at a puncture position in Fig. 13.
Fig. 15 is a cross-sectional view illustrating the time of puncture (puncture position) with a puncture needle.
Fig. 16 is a cross-sectional view illustrating a state where the housing is separated from the skin at the time of puncture by the puncture device illustrated in Fig. 15.

### Description of Embodiments

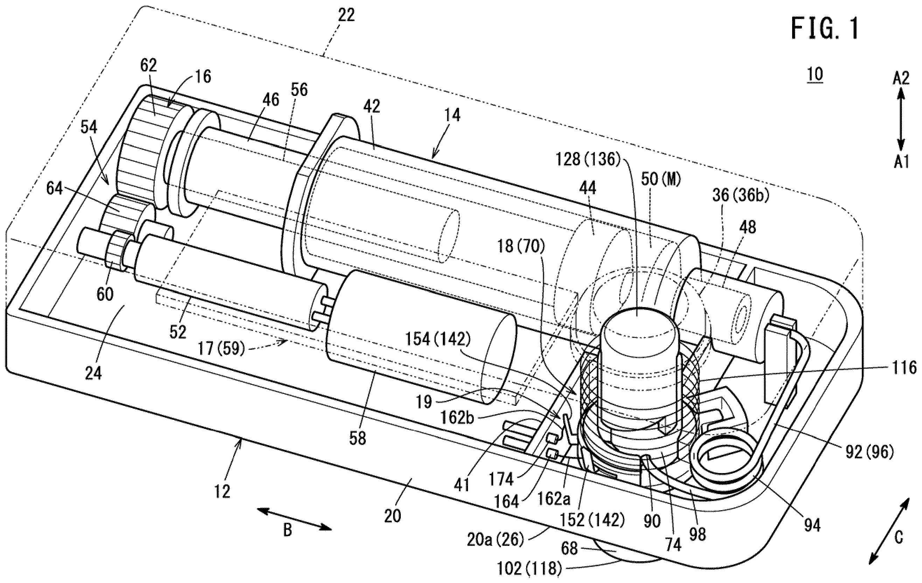

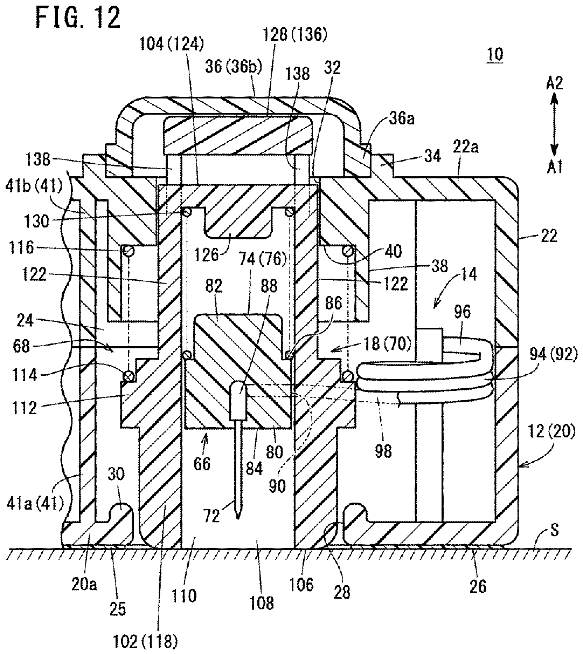

As illustrated in Fig. 1, a liquid medicine administration device 10 according to the present embodiment is brought into contact with skin S (refer to Fig. 12) of a patient (living body), for example, and is used to administer a liquid medicine M subcutaneously to the patient.

As illustrated in Fig. 1, the liquid medicine administration device 10 includes a housing 12, a prefilled syringe 14 that stores the liquid medicine M, a movement mechanism 16 that delivers the liquid medicine M in the prefilled syringe 14, a controller 17 that controls a drive unit 52 of the movement mechanism 16, and a puncture device 18 that can puncture the skin S (refer to Fig. 12) of the patient.

The housing 12 includes a housing main body 20 and a housing cover 22. When the liquid medicine administration device 10 including the puncture device 18 administers the liquid medicine M, the housing main body 20 is disposed on the skin S of the patient, and the housing cover 22 is disposed on an opposite side to the skin S with the housing main body 20 therebetween (refer to Fig. 12). The housing main body 20 and the housing cover 22 overlap in a thickness direction (arrow A1 and A2 directions) of the housing 12. The arrow A1 direction is a direction toward the skin S when the liquid medicine administration device 10 is installed in the skin S of the patient. The arrow A2 direction is a direction away from the skin S when the liquid medicine administration device 10 is installed in the skin S of the patient. Hereinafter, for convenience of description, a direction from the housing cover 22 toward the housing main body 20 (arrow A1 direction) is referred to as downward, and a direction from the housing main body 20 toward the housing cover 22 (arrow A2 direction) is referred to as upward.

In a top view of the liquid medicine administration device 10, the housing 12 has a rectangular shape elongated in a longitudinal direction (arrow B direction). The housing cover 22 covers an upper portion of the housing main body 20. The housing main body 20 and the housing cover 22 can be separated in a vertical direction (arrow A1 and A2 directions in Fig. 1).

The housing 12 has a space 24 surrounded by the housing main body 20 and the housing cover 22, and the prefilled syringe 14, the movement mechanism 16, a substrate 59 including the controller 17, and the puncture device 18 are housed in the space 24 of the housing 12 illustrated in Fig. 1.

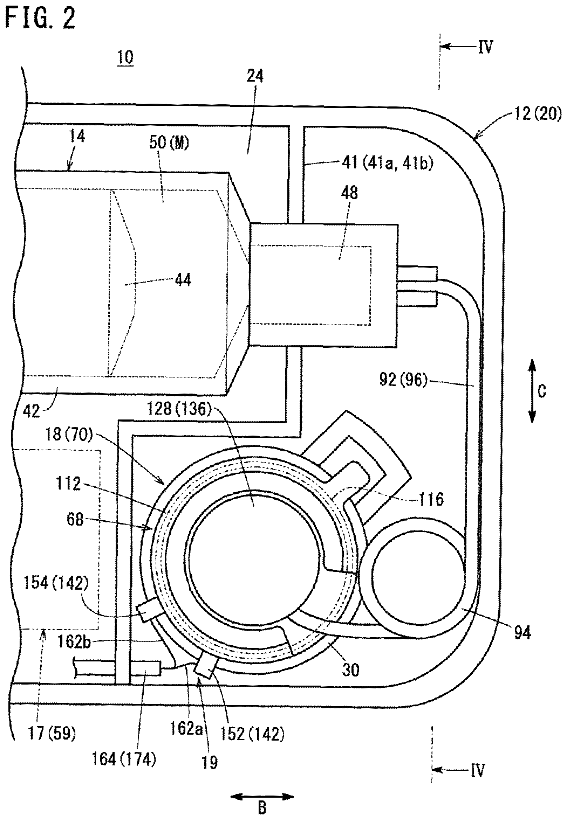

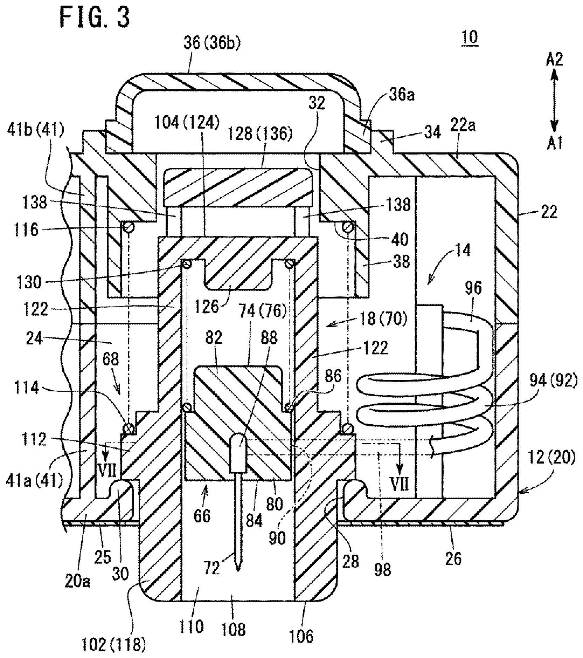

As illustrated in Fig. 3, a bottom wall 20a of the housing main body 20 has a contact surface 26 having contact with the skin S of the patient. In the present embodiment, the housing 12 has a bonding material 25 fixed to the bottom wall 20a of the housing main body 20, and one surface of the bonding material 25 is the contact surface 26. The contact surface 26 is flat along the longitudinal direction (arrow B direction in Fig. 2) of the housing 12 and a width direction (arrow C direction in Fig. 2) orthogonal to the longitudinal direction. The bottom wall 20a has a first hole portion 28. The first hole portion 28 is disposed at a distal portion along the longitudinal direction of the housing main body 20. The first hole portion 28 passes through the bottom wall 20a and communicates the outside of the housing main body 20 with the space 24.

The bottom wall 20a of the housing main body 20 has a stopper portion 30. The stopper portion 30 protrudes from the bottom wall 20a toward the housing cover 22 and is formed in an annular shape surrounding the first hole portion 28. That is, the stopper portion 30 is disposed on an outer periphery of the first hole portion 28.

An upper wall 22a of the housing cover 22 is formed in parallel with the bottom wall 20a of the housing main body 20. The upper wall 22a of the housing cover 22 has a second hole portion 32. The second hole portion 32 is disposed at a distal portion of the housing cover 22. In the thickness direction of the housing 12, the first hole portion 28 and the second hole portion 32 face each other. The upper wall 22a has an annular projection 34 surrounding the second hole portion 32. The annular projection 34 protrudes upward (arrow A2 direction) from the upper wall 22a. The annular projection 34 is disposed to be separated radially outward from the second hole portion 32.

On the upper wall 22a of the housing cover 22, a cover member 36 that is elastically deformable is disposed. The cover member 36 has a cap-like shape. The cover member 36 is disposed in the annular projection 34. The cover member 36 has an outer edge portion 36a and a covering portion 36b disposed on an inner side of the outer edge portion 36a. The outer edge portion 36a abuts on the upper wall 22a of the housing cover 22 adjacent to an inner peripheral surface of the annular projection 34.

The covering portion 36b offsets in an axial direction (arrow A2 direction) with respect to the outer edge portion 36a and faces the second hole portion 32. The covering portion 36b is disposed to be separated upward (arrow A2 direction) from the upper wall 22a of the housing cover 22. The covering portion 36b protrudes upward (arrow A2 direction) with respect to the annular projection 34. The second hole portion 32 is covered with the cover member 36.

The upper wall 22a of the housing cover 22 has a support portion 38. The support portion 38 has a cylindrical shape that protrudes from the upper wall 22a toward the housing main body 20 and surrounds the second hole portion 32. The second hole portion 32 passes through from the upper wall 22a to a lower end of the support portion 38. An inner peripheral surface of the support portion 38 has a housing-side receiving portion 40 recessed radially outward. The housing-side receiving portion 40 is a step formed from the lower end of the support portion 38 toward the upper wall 22a of the housing cover 22.

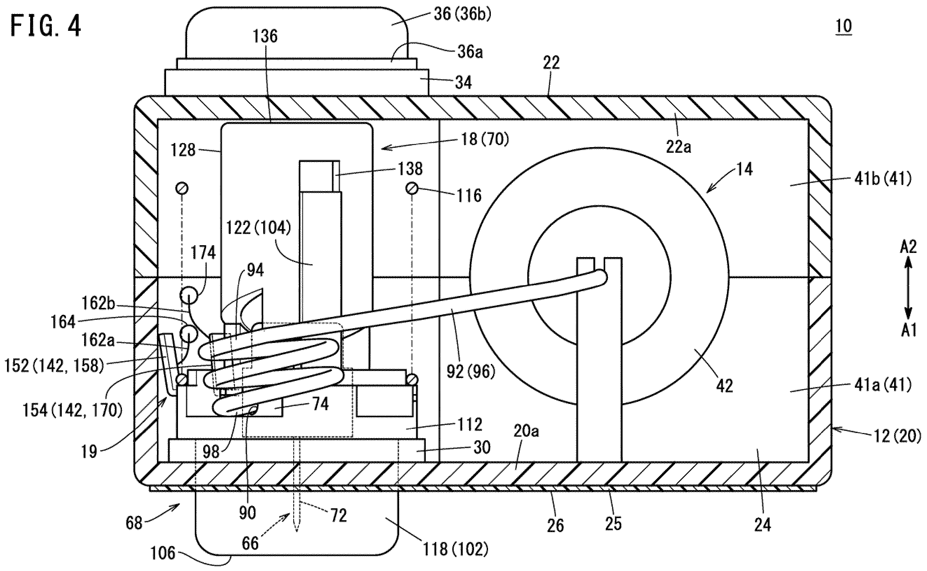

A distal portion of the housing 12 has a partition wall 41. As illustrated in Fig. 4, the partition wall 41 is configured by connecting a wall portion 41a erected from the bottom wall 20a toward the housing cover 22 and a wall portion 41b erected from the housing cover 22 toward the housing main body 20. The space 24 of the housing 12 is separated by the partition wall 41.

As illustrated in Fig. 1, the prefilled syringe 14 is housed in the housing 12 and is disposed along the longitudinal direction (arrow B direction) of the housing 12. The prefilled syringe 14 includes a cylindrical body 42, a gasket 44, and a plunger 46. The cylindrical body 42 has a cylindrical shape along an axial direction, of which a proximal end is opened. A distal end of the cylindrical body 42 has a nozzle 48 that protrudes in a distal direction. The gasket 44 is movably housed in the cylindrical body 42. The gasket 44 is made of an elastic material and is liquid-tightly inserted into the cylindrical body 42. A medicine chamber 50 filled with the liquid medicine M is provided between the distal end of the cylindrical body 42 and the gasket 44.

The plunger 46 is formed in a cylindrical shape. A distal portion of the plunger 46 is inserted into the cylindrical body 42 and is coupled to a proximal end of the gasket 44. A proximal portion of the plunger 46 is disposed outside the cylindrical body 42. The plunger 46 is disposed to be movable in the axial direction (the distal direction, a proximal direction, and the arrow B direction) relative to the cylindrical body 42.

The movement mechanism 16 includes the drive unit 52, a gear portion 54, and a shaft 56. The drive unit 52 is a motor that is rotationally driven by being supplied with power. The drive unit 52 is disposed in parallel with the prefilled syringe 14 in the housing 12. The drive unit 52 is electrically connected to a power supply unit 58. The power supply unit 58 is disposed in a distal direction of the drive unit 52. The power supply unit 58 can supply power to the drive unit 52. The power is supplied from the power supply unit 58 to the drive unit 52, and the power rotationally drives a driving shaft (not illustrated) of the drive unit 52.

As illustrated in Fig. 1, the controller 17 is disposed on the substrate 59. The substrate 59 is disposed along the longitudinal direction (arrow B direction) of the housing 12 in the space 24. The substrate 59 is disposed in parallel with the bottom wall 20a of the housing main body 20 and the housing cover 22. The substrate 59 covers the drive unit 52. The drive unit 52 is controlled by the controller 17.

As illustrated in Fig. 1, the gear portion 54 is disposed in a proximal direction of the drive unit 52. The gear portion 54 includes a drive gear 60 coupled to the driving shaft, a driven gear 62 coupled to the shaft 56, and an intermediate gear 64 meshed with the drive gear 60 and the driven gear 62. The intermediate gear 64 is disposed between the drive gear 60 and the driven gear 62. The drive gear 60, the driven gear 62, and the intermediate gear 64 are arranged in parallel in the width direction (arrow C direction) of the housing 12.

By rotationally driving the drive unit 52, the drive gear 60 rotates together with the driving shaft of the drive unit 52. As the drive gear 60 rotates, the intermediate gear 64 and the driven gear 62 rotate so that the shaft 56 rotates.

The shaft 56 extends along the axial direction and is inserted into the plunger 46. Inside the plunger 46, an outer peripheral surface of the shaft 56 and an inner peripheral surface of the plunger 46 are screwed together. The shaft 56 is housed in the cylindrical body 42 of the prefilled syringe 14 together with the plunger 46. The driven gear 62 is coupled to a proximal end of the shaft 56.

When a driving force of the drive unit 52 is transmitted to the shaft 56 via the gear portion 54 so as to rotate the shaft 56, the plunger 46 screwed with the shaft 56 moves along the axial direction (arrow B direction). The movement of the plunger 46 moves the gasket 44 in the cylindrical body 42 along the axial direction together with the plunger 46, and the liquid medicine M in the medicine chamber 50 is delivered to the distal direction by the gasket 44.

As illustrated in Fig. 2, the puncture device 18 is disposed at the distal portion of the housing 12. The puncture device 18 is disposed in the distal direction with respect to the partition wall 41 in the housing 12. Inside the housing 12, the puncture device 18 and the movement mechanism 16 are separated by the partition wall 41. As illustrated in Fig. 3, the puncture device 18 faces the first and second hole portions 28 and 32.

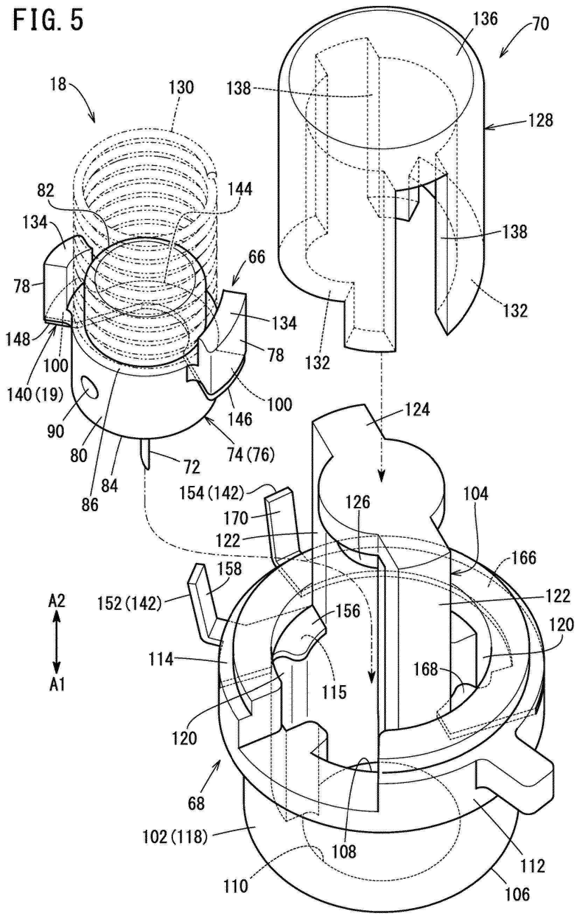

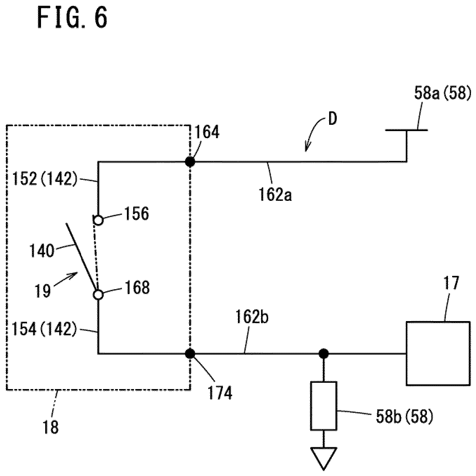

As illustrated in Fig. 5, the puncture device 18 includes a needle member 66, an abutting member 68, and a puncture mechanism 70 that biases the needle member 66 toward the skin S (refer to Fig. 12) of the patient. As illustrated in Fig. 6, the puncture device 18 constitutes a circuit D with the controller 17. The liquid medicine administration device 10 includes a connection unit 19 that switches between connection and disconnection of the circuit D. The connection unit 19 connects the circuit D at one position (in the present embodiment, a non-puncture position) of a puncture position at which a puncture needle 72 has moved toward the skin S and punctures the skin S and the non-puncture position at which the puncture needle 72 does not puncture the skin S. The connection unit 19 disconnects the circuit D when the puncture needle 72 is at the other position (in the present embodiment, the puncture position) of the puncture position and the non-puncture position.

As illustrated in Fig. 6, the circuit D includes the controller 17, a positive power supply 58a and a negative power supply 58b of the power supply unit 58, first and second connection members 164 and 174, the connection unit 19, and lead wires 162a and 162b. The positive power supply 58a and the connection unit 19 are electrically connected to each other by the lead wire 162a and the first connection member 164. The negative power supply 58b, the controller 17, and the connection unit 19 are electrically connected to each other by the lead wire 162b and the second connection member 174. As illustrated in Fig. 4, the first and second connection members 164 and 174 are each made of a metal material and held by the partition wall 41 of the housing 12.

As illustrated in Fig. 5, the needle member 66 includes the puncture needle 72 and a needle hub 74 that holds the puncture needle 72. The puncture needle 72 is a hollow needle. A distal end of the puncture needle 72 can puncture the skin S of the patient. A proximal end of the puncture needle 72 is held at an axial center of the needle hub 74.

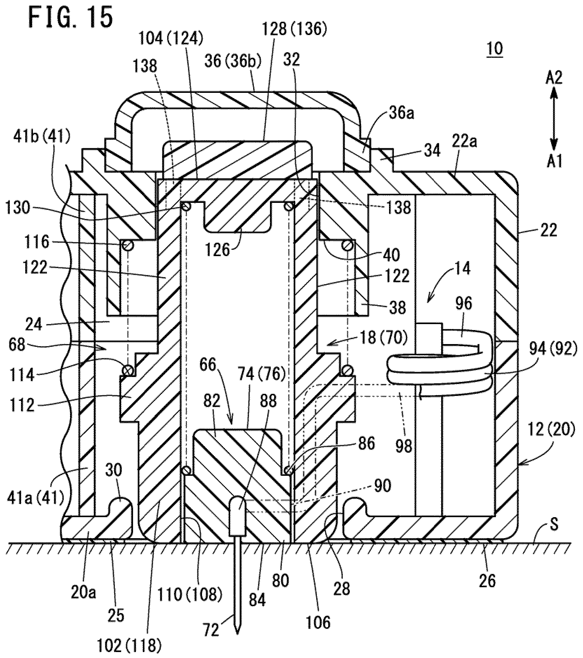

The needle hub 74 includes a hub main body 76. The hub main body 76 has a circular shape as viewed from an axial direction of the needle hub 74. The hub main body 76 includes a large diameter portion 80 and a small diameter portion 82. The large diameter portion 80 is disposed in the distal direction (arrow A1 direction) of the hub main body 76. A distal end of the hub main body 76 (large diameter portion 80) has a hub surface 84. The hub surface 84 is a flat surface orthogonal to an axis of the needle hub 74. The proximal end of the puncture needle 72 is held at an axial center of the hub surface 84. When the skin S of the patient is punctured by the needle member 66, the hub surface 84 is a surface having contact with the skin S (refer to Fig. 15).

The small diameter portion 82 is disposed in a proximal direction (arrow A2 direction) of the large diameter portion 80. A diameter of the small diameter portion 82 is smaller than a diameter of the large diameter portion 80. At a boundary between the small diameter portion 82 and the large diameter portion 80, an annular hub-side receiving portion 86 extending in a radial direction is provided.

As illustrated in Fig. 3, the large diameter portion 80 of the hub main body 76 has a communication path 88. A distal end of the communication path 88 extends along an axial center of the hub main body 76 and is opened in the hub surface 84. The distal end of the communication path 88 communicates with the inside of the puncture needle 72. A proximal end of the communication path 88 extends outward in the radial direction and is opened in an outer peripheral surface of the large diameter portion 80. The outer peripheral surface of the large diameter portion 80 has a connection hole 90 (refer to Fig. 5), and the connection hole 90 and the proximal end of the communication path 88 communicate with each other.

As illustrated in Fig. 4, the connection hole 90 of the needle hub 74 and the prefilled syringe 14 are connected with a tube 92 that is elastically deformable. The tube 92 is a tubular body and is disposed at the distal portion of the housing 12 (refer to Fig. 2). The tube 92 has flexibility and can deliver the liquid medicine M from the prefilled syringe 14 to the needle member 66. The liquid medicine M is supplied from the prefilled syringe 14 to the communication path 88 of the needle member 66 (refer to Fig. 3) through the tube 92 and is delivered from the communication path 88 to the puncture needle 72. The tube 92 includes a spirally wound spiral portion 94, a first extending portion 96 that extends from one end of the spiral portion 94, and a second extending portion 98 that extends from another end of the spiral portion 94.

The spiral portion 94 has a substantially circular shape and wound along the vertical direction (arrow A1 and A2 directions) a plurality of times. As illustrated in Fig. 2, the first extending portion 96 substantially linearly extends along the width direction of the housing 12. An end portion of the first extending portion 96 is connected to the nozzle 48 of the prefilled syringe 14. The second extending portion 98 extends along the longitudinal direction of the housing 12. As illustrated in Fig. 4, an end portion of the second extending portion 98 is connected to the connection hole 90 of the needle hub 74. The spiral portion 94 of the tube 92 is housed in the housing 12 in a state of being bent in the vertical direction. When the needle member 66 is displaced in the axial direction (arrow A1 and A2 directions) of the needle member 66 with respect to the housing 12, the spiral portion 94 of the tube 92 is elastically deformed as the second extending portion 98 moves together with the needle member 66.

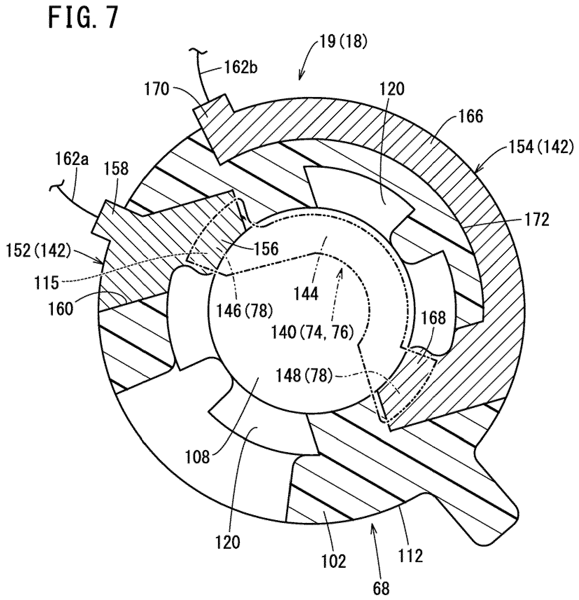

As illustrated in Fig. 5, the needle hub 74 further includes a pair of guided portions 78 disposed on a radially outer side of the hub main body 76. A distal end of the guided portion 78 is connected to the outer peripheral surface of the large diameter portion 80. The pair of guided portions 78 faces an outer peripheral surface of the small diameter portion 82 and is disposed to be separated radially outward from the outer peripheral surface of the small diameter portion 82. The guided portion 78 on one side and the guided portion 78 on the other side are symmetrically disposed with respect to the axial center of the hub main body 76. Each of the guided portions 78 includes a locking portion 100. The locking portion 100 is disposed at the distal end of the guided portion 78. The locking portion 100 has a flat shape orthogonal to the axial direction of the needle hub 74. As illustrated in Fig. 7, on a cross section perpendicular to the axial direction of the needle member 66, a cross-sectional shape of the guided portion 78 is an arc shape.

As illustrated in Fig. 3, the abutting member 68 is provided in the housing 12 to be movable along the thickness direction (arrow A1 and A2 directions) of the housing 12. The abutting member 68 is disposed in the first hole portion 28 of the housing main body 20. As illustrated in Fig. 5, the abutting member 68 includes a cylindrical portion 102 and a gate portion 104 that protrudes from the cylindrical portion 102 in the proximal direction (arrow A2 direction).

The cylindrical portion 102 is disposed at a distal portion of the abutting member 68. A distal end of the cylindrical portion 102 has an abutting surface 106 that can have contact with the skin S of the patient (refer to Fig. 12). The abutting surface 106 is a flat surface orthogonal to an axis of the abutting member 68. As illustrated in Fig. 3, a housing space 108 in which the needle member 66 is movably housed is provided in the cylindrical portion 102. The needle member 66 can move in the axial direction (arrow A1 and A2 directions) relative to the abutting member 68, through the housing space 108.

The housing space 108 has an opening 110 that is opened at the distal end (abutting surface 106) of the cylindrical portion 102. The puncture needle 72 can protrude from the abutting surface 106 through the opening 110.

A proximal end of the cylindrical portion 102 has a flange portion 112 of which a diameter is expanded radially outward. The flange portion 112 has an annular shape surrounding the housing space 108. As illustrated in Fig. 5, the flange portion 112 has a spring receiving portion 114 having an annular shape. The spring receiving portion 114 is formed in a stepped shape recessed radially inward from an outer peripheral surface of the flange portion 112.

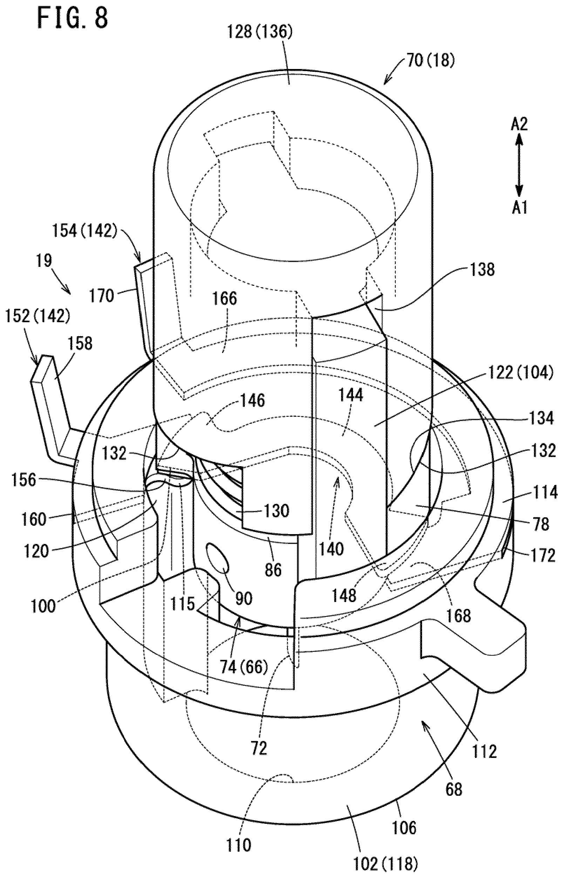

The abutting member 68 includes an engagement portion 115. The engagement portion 115 is a flat surface orthogonal to the axial direction of the abutting member 68. As illustrated in Fig. 8, in an initial state of the puncture device 18, the locking portion 100 of the needle hub 74 is locked to the engagement portion 115 of the abutting member 68 via a first contact portion 146 and a second contact portion 148 of a first circuit component 140 to be described later. That is, the first and second contact portions 146 and 148 abut on the engagement portion 115. As a result, the needle hub 74 is prevented from moving in a puncture direction (the distal direction and the arrow A1 direction) with respect to the abutting member 68.

As illustrated in Fig. 3, a following biasing member 116 is disposed between the cylindrical portion 102 of the abutting member 68 and the support portion 38 of the housing 12 in the housing 12. The following biasing member 116 is a spring member including a coil spring. The following biasing member 116 is disposed between the housing-side receiving portion 40 of the support portion 38 and the spring receiving portion 114 of the cylindrical portion 102. The small diameter portion 82 of the hub main body 76 is inserted into one end portion of the puncture biasing member 130. A projection 126 to be described later of the abutting member 68 is inserted into the other end portion of the puncture biasing member 130. A biasing force of the following biasing member 116 is biased against the abutting member 68. The abutting member 68 is biased in the distal direction (the puncture direction and the arrow A1 direction) by the biasing force of the following biasing member 116. That is, the following biasing member 116 biases the abutting member 68 toward the skin S of the patient.

When the abutting surface 106 of the abutting member 68 does not have contact with the skin S of the patient, the flange portion 112 of the abutting member 68 abuts on the stopper portion 30. The flange portion 112 of the abutting member 68 abuts on the stopper portion 30 of the housing 12 so as to prevent removal of the abutting member 68 through the first hole portion 28.

The abutting member 68 has a protruding portion 118 that protrudes from the contact surface 26 of the housing 12 when the abutting surface 106 of the abutting member 68 does not have contact with the skin S. The protruding portion 118 protrudes to the outside of the housing 12 through the first hole portion 28 of the housing main body 20. When the contact surface 26 of the housing 12 has contact with the skin S of the patient, the abutting surface 106 of the abutting member 68 is pressed by the skin S so that the protruding portion 118 is housed in the housing 12 through the first hole portion 28 (refer to Fig. 12).

As illustrated in Fig. 5, the cylindrical portion 102 has a pair of guide grooves 120 that can guide the needle member 66 in the axial direction. A proximal end of the guide groove 120 is disposed at a position adjacent to the engagement portion 115 in a circumferential direction of the cylindrical portion 102. The guide groove 120 is recessed radially outward from an inner peripheral surface of the cylindrical portion 102 and faces the housing space 108. The guided portions 78 of the needle member 66 can be inserted into the guide grooves 120, respectively. Each of the guide grooves 120 extends along the axial direction (arrow A1 and A2 directions) of the abutting member 68.

The gate portion 104 extends from the proximal end of the cylindrical portion 102 in the proximal direction (arrow A2 direction). The gate portion 104 includes a pair of column portions 122 and a bridge portion 124 that bridges proximal ends of the column portions 122. The pair of column portions 122 extends along the axial direction from the cylindrical portion 102 toward the proximal direction, and is symmetrically disposed with respect to an axial center of the cylindrical portion 102.

The bridge portion 124 is orthogonal to the axial direction of the abutting member 68 and connects a proximal end of the column portion 122 on one side and a proximal end of the column portion 122 on the other side. The projection 126 that protrudes toward the cylindrical portion 102 is provided at the center of the bridge portion 124. In the axial direction of the abutting member 68, the bridge portion 124 and the needle member 66 face each other (refer to Fig. 3).

The puncture mechanism 70 causes the needle member 66 to protrude from the abutting surface 106 of the abutting member 68, in accordance with an operation of a user (for example, patient or medical personnel). As illustrated in Fig. 5, the puncture mechanism 70 includes a pusher 128 in which a pair of first inclined portions 132 is provided, a puncture biasing member 130 to be described later, and a pair of second inclined portions 134 provided on the needle hub 74 and abutting on the first inclined portion 132.

As illustrated in Fig. 3, the pusher 128 is movably disposed in the puncture direction (axial direction and arrow A1 direction) of the puncture needle 72 with respect to the housing 12. The pusher 128 is disposed in the proximal direction (arrow A2 direction) of the abutting member 68. The pusher 128 has a bottomed cylindrical shape. The gate portion 104 of the abutting member 68 is inserted into the pusher 128. A top portion of the pusher 128 has a pressing portion 136. In the initial state of the puncture device 18, the pressing portion 136 is disposed in the second hole portion 32 of the housing cover 22.

As illustrated in Fig. 8, an outer peripheral surface of the pusher 128 has a pair of slit grooves 138 symmetrically disposed with respect to an axial center of the pusher 128. The slit groove 138 extends along the axial direction from a distal end of the pusher 128 toward a proximal end. The slit groove 138 passes through the pusher 128 in a radial direction. The column portions 122 of the abutting member 68 are inserted into the slit grooves 138, respectively. In the initial state of the puncture device 18, the proximal end of the slit groove 138 and the proximal end of the column portion 122 of the abutting member 68 are separated in the axial direction. By inserting the column portions 122 of the gate portion 104 into the pair of slit grooves 138, the pusher 128 and the abutting member 68 can relatively move along the axial direction (arrow A1 and A2 directions). That is, the pusher 128 and the abutting member 68 are prevented from relatively rotating around the axes of the pusher 128 and the abutting member 68.

The puncture biasing member 130 is a spring member including a coil spring. The puncture biasing member 130 is disposed between the bridge portion 124 of the abutting member 68 (refer to Fig. 3) and the hub-side receiving portion 86 of the needle hub 74. A biasing force of the puncture biasing member 130 is biased against the needle hub 74. The puncture biasing member 130 biases the needle member 66 toward the skin S of the patient. By biasing the needle member 66 toward the skin S of the patient by the puncture biasing member 130, the needle member 66 can move in the puncture direction (arrow A1 direction) relative to the abutting member 68.

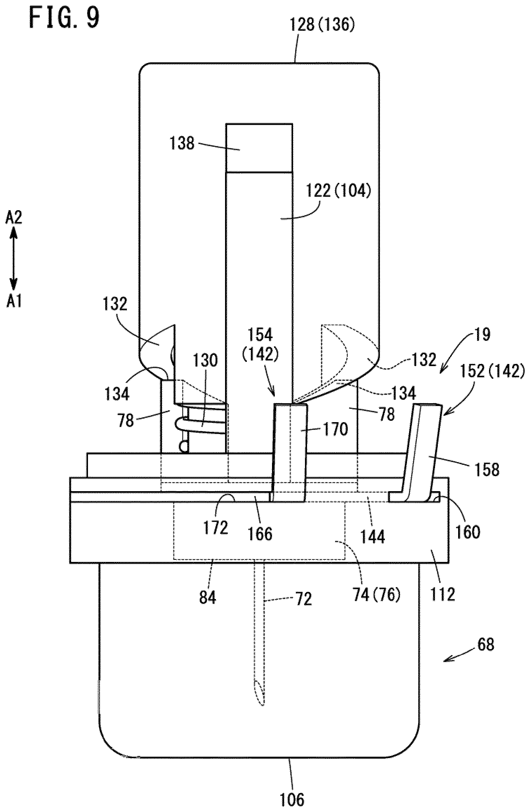

As illustrated in Fig. 9, the pair of first inclined portions 132 is disposed at the distal end of the pusher 128. The first inclined portion 132 is inclined with respect to the axial direction of the pusher 128. In other words, the first inclined portion 132 is inclined with respect to the puncture direction (arrow A1 direction) of the puncture needle 72. The first inclined portion 132 is inclined so as to extend clockwise toward the distal direction (arrow A1 direction) when the pusher 128 is viewed from the proximal end toward the distal end.

The second inclined portion 134 is disposed in each of the guided portions 78 of the needle hub 74. The second inclined portion 134 is disposed at a proximal end of the guided portion 78. The first inclined portion 132 of the pusher 128 can abut on the second inclined portion 134. The second inclined portion 134 is inclined with respect to the puncture direction (arrow A1 direction) of the needle member 66. That is, the second inclined portion 134 is inclined so as to extend clockwise toward the distal direction (arrow A1 direction) when the needle member 66 is viewed from the proximal end toward the distal direction.

The second inclined portion 134 is inclined in the same direction as the first inclined portion 132. Therefore, in the initial state of the puncture device 18 illustrated in Fig. 8, the first inclined portion 132 and the second inclined portion 134 come into surface contact with each other.

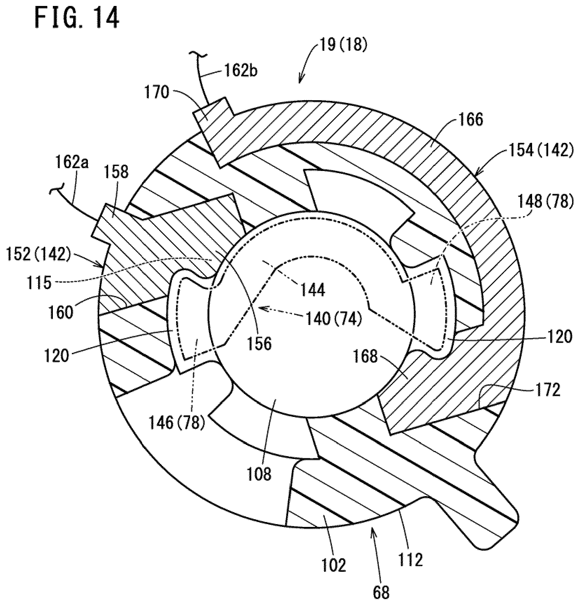

As illustrated in Fig. 7, the connection unit 19 connects the circuit D (refer to Fig. 6) at the non-puncture position where the puncture needle 72 does not puncture the skin S. The connection unit 19 disconnects the circuit D when the puncture needle 72 is at the puncture position where the skin S is punctured (refer to Fig. 14). As illustrated in Figs. 7 and 8, the connection unit 19 includes the first circuit component 140 and a second circuit component 142.

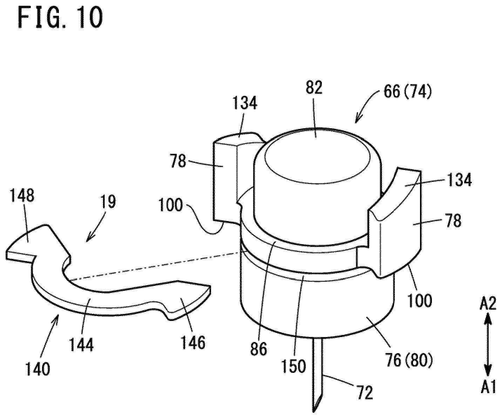

The first circuit component 140 is formed in a plate shape using a metal material and is disposed on the needle hub 74 of the needle member 66. As illustrated in Fig. 10, the first circuit component 140 includes a first arcuate portion 144 and a pair of first and second contact portions 146 and 148 disposed at both ends of the first arcuate portion 144.

As illustrated in Fig. 7, the first arcuate portion 144 is formed in a substantially arcuate shape and is disposed on the hub main body 76 of the needle hub 74. The first arcuate portion 144 is curved with substantially the same radius as that of an outer peripheral surface of the needle hub 74. The first arcuate portion 144 is inserted into a first mounting hole 150 opened in the outer peripheral surface of the needle hub 74. The first arcuate portion 144 is inserted radially inward into the first mounting hole 150. The first circuit component 140 including the first arcuate portion 144 is held by the needle hub 74 through the first mounting hole 150. When the first arcuate portion 144 is inserted into the first mounting hole 150, the first arcuate portion 144 does not protrude radially outward from the outer peripheral surface of the needle hub 74. The first circuit component 140 is disposed to be orthogonal to the axial direction of the needle hub 74.

The pair of first and second contact portions 146 and 148 is disposed at one end and the other end of the first arcuate portion 144 in an extending direction. The first and second contact portions 146 and 148 protrude radially outward from the first arcuate portion 144. As illustrated in Fig. 5, the first and second contact portions 146 and 148 are disposed on the locking portions 100 of the pair of guided portions 78, respectively, of the needle hub 74. That is, the first and second contact portions 146 and 148 are disposed radially outward from an outer peripheral surface of the hub main body 76. When viewed from the axial direction of the needle hub 74, the first and second contact portions 146 and 148 are formed in substantially the same shape as that of the guided portion 78 (refer to Fig. 7). The first and second contact portions 146 and 148 are disposed at the distal ends of the guided portions 78 and cover the locking portions 100.

As illustrated in Fig. 5, the second circuit component 142 is formed in a plate shape using a metal material, and is disposed on the cylindrical portion 102 of the abutting member 68. The second circuit component 142 includes a first terminal member 152 and a second terminal member 154.

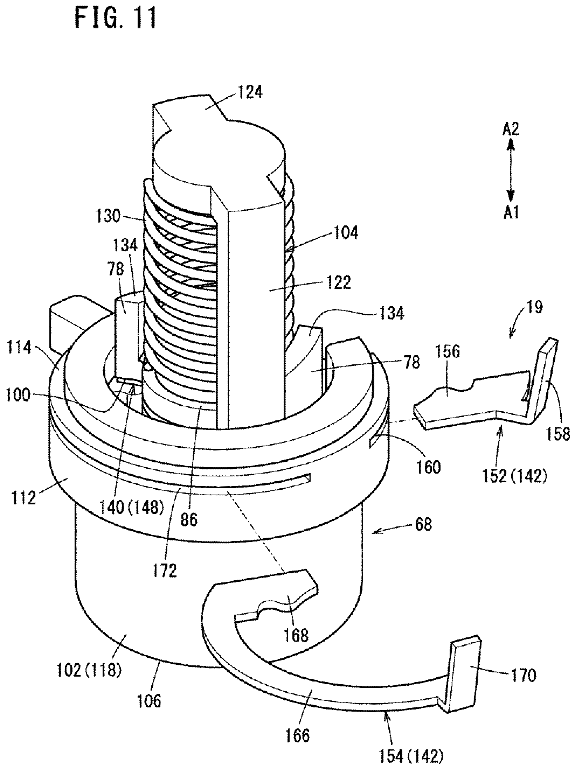

As illustrated in Fig. 11, the first terminal member 152 is disposed on the flange portion 112 of the cylindrical portion 102. The first terminal member 152 includes a first contacted portion 156 and a first terminal 158.

The first contacted portion 156 can have contact with the first contact portion 146 and is disposed in a second mounting hole 160 opened in an outer peripheral surface of the cylindrical portion 102. The first contacted portion 156 is orthogonal to the axial direction of the abutting member 68. As illustrated in Fig. 7, the first contacted portion 156 is exposed to the inside of the cylindrical portion 102 through the second mounting hole 160. The first contacted portion 156 is disposed on the engagement portion 115 of the cylindrical portion 102 and covers a part of the engagement portion 115. The first contacted portion 156 does not protrude radially outward from the outer peripheral surface of the cylindrical portion 102. At the non-puncture position before the puncture needle 72 punctures the skin S, the first contacted portion 156 and the first contact portion 146 of the first circuit component 140 face each other and abut on each other.

As illustrated in Fig. 8, the first terminal 158 is disposed at an outer edge portion of the first contacted portion 156. The first terminal 158 is bent and extends with respect to the first contacted portion 156. The first terminal 158 is separated radially outward from the flange portion 112 of the cylindrical portion 102. The first terminal 158 is substantially parallel to the axial direction of the cylindrical portion 102. The first terminal 158 extends toward the bridge portion 124 of the abutting member 68. The first terminal 158 is electrically connected to the first connection member 164 by the lead wire 162a. The substrate 59 and the positive power supply 58a of the power supply unit 58 are electrically connected to the first terminal member 152 (refer to Fig. 6).

The second terminal member 154 can have contact with the second contact portion 148 and is disposed on the flange portion 112 of the cylindrical portion 102. The second terminal member 154 and the first terminal member 152 are disposed to be separated from each other in the circumferential direction of the cylindrical portion 102. As illustrated in Fig. 11, the second terminal member 154 includes a second arcuate portion 166, a second contacted portion 168, and a second terminal 170.

As illustrated in Fig. 7, the second arcuate portion 166 is inserted into a third mounting hole 172 formed in an arcuate shape and opened in the outer peripheral surface of the cylindrical portion 102. The second arcuate portion 166 is housed in the cylindrical portion 102 through the third mounting hole 172. The second arcuate portion 166 does not protrude radially outward from the outer peripheral surface of the cylindrical portion 102.

The second contacted portion 168 can have contact with the second contact portion 148 and is disposed at one end of the second arcuate portion 166 in an extending direction. The second contacted portion 168 protrudes radially inward from the second arcuate portion 166. The second contacted portion 168 is inserted into the third mounting hole 172 together with the second arcuate portion 166. The second contacted portion 168 is orthogonal to the axial direction of the abutting member 68. The second contacted portion 168 is exposed to the inside of the cylindrical portion 102 through the third mounting hole 172. The second contacted portion 168 is disposed on the engagement portion 115 of the cylindrical portion 102 and covers a part of the engagement portion 115. At the non-puncture position before the puncture needle 72 punctures the skin S, the second contacted portion 168 and the second contact portion 148 of the first circuit component 140 face each other and abut on each other. The circuit D is connected by the contact between the second circuit component 142 and the first circuit component 140 (refer to the two-dot chain line shape in Fig. 6).

The second terminal 170 is disposed at the other end of the second arcuate portion 166 in the extending direction. The second terminal 170 is disposed at an outer edge portion of the second arcuate portion 166. The second terminal 170 is bent and extends with respect to the second arcuate portion 166 and the second contacted portion 168. The second terminal 170 is separated radially outward from the flange portion 112 of the cylindrical portion 102. The second terminal 170 is parallel to the axial direction of the cylindrical portion 102. The second terminal 170 extends toward the bridge portion 124 of the abutting member 68. The second terminal 170 is electrically connected to the second connection member 174 by the lead wire 162b. The substrate 59 and the negative power supply 58b of the power supply unit 58 are electrically connected to the second terminal member 154 (refer to Fig. 6). That is, the connection unit 19 is disposed between the needle member 66 and the abutting member 68 at the non-puncture position where the puncture needle 72 does not puncture the skin S.

Next, an operation of the liquid medicine administration device 10 will be described. A power supply (not illustrated) of the liquid medicine administration device 10 is turned on by an operator (not illustrated). Power is supplied from the power supply unit 58 illustrated in Fig. 1 to the substrate 59.

First, in the liquid medicine administration device 10 before use (in the initial state), illustrated in Fig. 3, the distal portion (protruding portion 118) of the abutting member 68 including the abutting surface 106 protrudes toward the contact surface 26 (bonding material 25) of the housing 12.

At this time, as illustrated in Fig. 7, the first contact portion 146 of the first circuit component 140 faces and has contact with the first contacted portion 156 of the first terminal member 152 of the second circuit component 142. The second contact portion 148 of the first circuit component 140 faces and has contact with the second contacted portion 168 of the first terminal member 152 of the second circuit component 142. As a result, the first terminal member 152 and the second terminal member 154 are electrically connected by the first circuit component 140 to obtain a connection state of the circuit D (refer to the two-dot chain line shape in Fig. 6). The circuit D including the lead wires 162a and 162b, the first and second connection members 164 and 174, and the controller 17 of the substrate 59 is connected. With the connection of the circuit D, the controller 17 detects the non-puncture position where the puncture needle 72 does not puncture the skin S.

As illustrated in Fig. 12, the contact surface 26 of the housing 12 is brought into contact with the skin S of the patient (the bonding material 25 is attached to the skin S), whereby the abutting surface 106 of the abutting member 68 has contact with the skin S, and the protruding portion 118 is pushed toward the direction (arrow A2 direction) of the housing 12 by the skin S. The abutting member 68 moves in the axial direction toward the direction of the housing 12 while compressing the following biasing member 116, and the protruding portion 118 is housed in the space 24 of the housing 12 through the first hole portion 28.

At this time, the needle member 66 and the pusher 128 move with respect to the housing 12, together with the abutting member 68. A positional relationship between the abutting member 68 and the needle member 66 is not changed by the biasing force of the puncture biasing member 130. A positional relationship between the abutting member 68 and the pusher 128 does not change.

The movement of the needle member 66 elastically deforms the spiral portion 94 of the tube 92 to be contracted upward in the axial direction.

As the abutting member 68 moves, the pusher 128 is pushed up by the needle member 66, and the pressing portion 136 of the pusher 128 is housed in the cover member 36 through the second hole portion 32. Since the pressing portion 136 protrudes upward (arrow A2 direction) from the upper wall 22a of the housing cover 22, the user can press the pressing portion 136 toward the puncture direction (arrow A1 direction) via the covering portion 36b of the cover member 36.

Next, the user pushes the pressing portion 136 of the pusher 128 via the cover member 36 and pushes the pressing portion 136 into the housing 12. As a result, the pusher 128 moves in the puncture direction (arrow A1 direction) along the support portion 38, and the first inclined portion 132 presses the second inclined portion 134 in the puncture direction. At this time, as illustrated in Fig. 8, the locking portion 100 abuts on the engagement portion 115, and the guided portion 78 having the second inclined portion 134 is prevented from moving downward. A position of the needle member 66 in a rotation direction with respect to the abutting member 68 in the initial state illustrated in Fig. 8 is referred to as a "first position".

Therefore, when the second inclined portion 134 of the needle hub 74 is pushed in the puncture direction (arrow A1 direction) by the first inclined portion 132 of the pusher 128, the pair of guided portions 78 receives a force counterclockwise by the inclination of the first inclined portion 132 and the second inclined portion 134. As a result, the needle hub 74 does not move in the puncture direction and starts to rotate in the counterclockwise direction from the first position, around the axis of the needle hub 74.

At this time, in Fig. 7, with the rotation of the needle hub 74, the first contact portion 146 starts to be separated from the first contacted portion 156 in the counterclockwise direction, and the second contact portion 148 starts to be separated from the second contacted portion 168 in the counterclockwise direction.

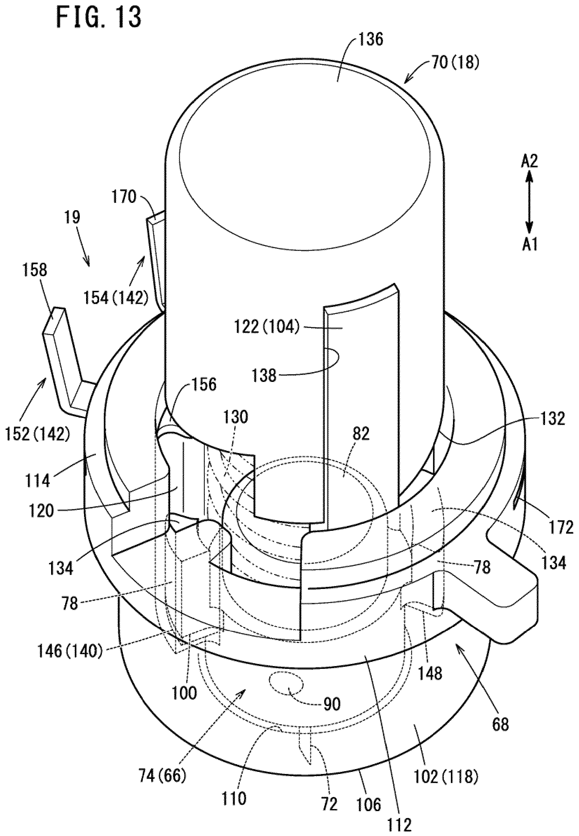

As illustrated in Figs. 13 and 14, when the needle hub 74 rotates, the pair of guided portions 78 reaches a second position facing the proximal ends of the guide grooves 120, and the locking portion 100 is removed from the engagement portion 115, the needle hub 74 moves in the puncture direction (arrow A1 direction) by the biasing force of the puncture biasing member 130. At this time, the guided portion 78 is inserted into the guide groove 120, the guided portion 78 is guided along the guide groove 120 in the puncture direction, and the needle hub 74 moves. A proximal end of the slit groove 138 of the pusher 128 abuts on the proximal end of the column portion 122 of the abutting member 68, and the pusher 128 is held in the puncture direction by the abutting member 68 (refer to Fig. 13).

As a result, as illustrated in Fig. 15, the needle hub 74 moves in the puncture direction (arrow A1 direction) with respect to the abutting member 68, the hub surface 84 abuts on the skin S, and the puncture needle 72 punctures the skin S of the patient. At this time, the tube 92 is elastically deformed downward along with the movement of the needle member 66 in the puncture direction.

At this time, as illustrated in Fig. 14, the first contact portion 146 is completely separated from the first contacted portion 156 to be in a non-contact state, and the second contact portion 148 is completely separated from the second contacted portion 168 to be in a non-contact state. Therefore, energization between the first terminal member 152 and the second terminal member 154 by the first circuit component 140 is cut off. As a result, the first circuit component 140 and the second circuit component 142 are separated from each other, so that the circuit D is in a disconnected state. The circuit D including the lead wires 162a and 162b, the first and second connection members 164 and 174, and the controller 17 of the substrate 59 is disconnected. When the circuit D is disconnected, the controller 17 detects the movement of the puncture needle 72 to the puncture position.

After the puncture of the puncture needle 72 with respect to the skin S is completed, the controller 17 detects the movement of the puncture needle 72 to the puncture position, and the controller 17 outputs a control signal to the drive unit 52. Power is supplied to the drive unit 52 to drive the drive unit 52. As the drive unit 52 is driven, each of the drive gear 60, the intermediate gear 64, and the driven gear 62 rotates, the shaft 56 rotates, so that the plunger 46 moves toward the distal direction in the cylindrical body 42. When the gasket 44 is pushed in the distal direction by the plunger 46, the liquid medicine M in the medicine chamber 50 is delivered from the nozzle 48 to the tube 92. The liquid medicine M is supplied to the communication path 88 of the needle hub 74 illustrated in Fig. 15, and is subcutaneously administered to the patient through the puncture needle 72.

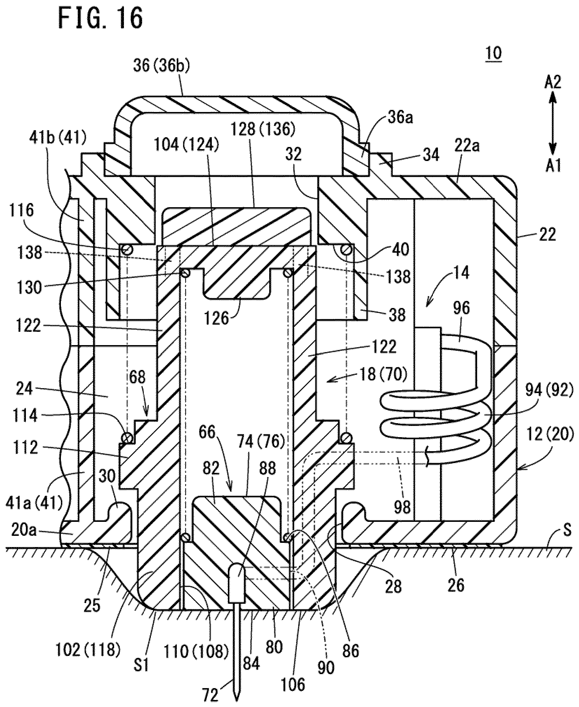

Fig. 16 illustrates a situation where, in a state where the contact surface 26 of the housing 12 has contact with the skin S of the patient and the skin S is punctured with the puncture needle 72, a facing portion S1 of the skin S facing the abutting member 68 and the needle member 66 and the contact surface 26 of the housing 12 are separated in the puncture direction (arrow A1 direction) of the needle member 66 due to a body movement of the patient or the like.

When the facing portion S1 of the skin S is separated from the contact surface 26 of the housing 12 in the puncture direction, the abutting member 68 biased by the biasing force of the following biasing member 116 moves relative to the housing 12 so as to maintain contact of the abutting surface 106 with the facing portion S1. As a result, the abutting surface 106 and the facing portion S1 are not separated in the puncture direction. As the abutting member 68 moves following the facing portion S1, the needle member 66 moves together with the abutting member 68, and the puncture of the puncture needle 72 to the skin S (facing portion S1) is maintained.

Note that the connection unit 19 is not limited to a case where the circuit D is connected at the non-puncture position of the puncture needle 72 and the circuit D is disconnected when the puncture needle 72 starts moving to the puncture position. The circuit D may be connected by bringing the second circuit component 142 (the first contact portion 146 and the second contact portion 148) disposed at the distal end of the guide groove 120 into contact with the first circuit component 140 at the puncture position of the puncture needle 72, the circuit D may be disconnected by separating the first circuit component 140 and the second circuit component 142 at the non-puncture position of the puncture needle 72, and the drive unit 52 may be controlled to move the plunger 46 toward the distal end to administer the liquid medicine M at the puncture position where the circuit D is connected.

As described above, in the embodiment of the present invention, the liquid medicine administration device 10 includes the connection unit 19 capable of connecting the circuit D at the non-puncture position where the puncture needle 72 does not puncture the skin S and disconnecting the circuit D at the puncture position. When the disconnection of the circuit D is detected as the puncture needle 72 moves to the puncture position, the controller 17 controls the drive unit 52 to move the plunger 46 of the prefilled syringe 14 toward the distal end to administer the liquid medicine M.

As a result, the plunger 46 can be moved toward the distal end by the drive unit 52 after the movement of the puncture needle 72 to the puncture position is detected. Therefore, at the non-puncture position where the puncture needle 72 does not puncture the skin S, the liquid medicine M is prevented from leaking from the puncture needle 72 due to the erroneous driving of the drive unit 52.

Since the connection unit 19 includes the first circuit component 140 disposed on the needle hub 74 and the second circuit component 142 disposed on the abutting member 68, the contact state and the non-contact state between the first circuit component 140 and the second circuit component 142 can be easily switched by relatively moving the needle hub 74 and the abutting member 68, and accordingly, the circuit D can be connected or disconnected.

When the puncture needle 72 punctures the skin S, the needle member 66 moves toward the skin S, and the first circuit component 140 and the second circuit component 142 are separated from each other to release the contact therebetween, whereby the circuit D is disconnected. As a result, the controller 17 can detect that the puncture needle 72 is not at the non-puncture position and the puncture needle 72 has moved toward the puncture position by disconnecting the circuit D.

In a case where a relative position between the facing portion S1, which faces the abutting surface 106, of the skin S and the contact surface 26 of the housing 12 changes due to the body movement of the patient or the like in a state where the contact surface 26 of the housing 12 has contact with the skin S, the abutting member 68 moves relative to the housing 12 so as to follow the facing portion S1 of the skin S. Therefore, removal of the puncture needle 72 of the needle member 66 from the skin S is prevented, and the abutting member 68 and the needle member 66 do not move relative to each other, so that the contact state or the non-contact state between the first circuit component 140 and the second circuit component 142 is suitably maintained.

Since an abutting state of the abutting surface 106 of the abutting member 68 with respect to the skin S is maintained by the following biasing member 116, by including the following biasing member 116 that biases the abutting member 68 toward the skin S in the housing 12, the removal of the needle member 66 (puncture needle 72) can be reliably prevented.

Since the puncture biasing member 130 that biases the needle member 66 toward the skin S is provided in the housing 12, the contact state between the first circuit component 140 and the second circuit component 142 can be easily and reliably switched by the puncture biasing member 130 for biasing the puncture needle 72 of the needle member 66 toward the skin S. Therefore, the contact state between the first circuit component 140 and the second circuit component 142 can be switched without increasing the number of components of the liquid medicine administration device 10. The puncture biasing member 130 can cause the puncture needle 72 of the needle member 66 to protrude and reliably puncture the skin S.

In the puncture device 18, the contact state between the first circuit component 140 disposed on the needle member 66 and the second circuit component 142 disposed on the abutting member 68 can be switched when the needle member 66 rotates from the first position to the second position.

Since the pusher 128 is housed in the housing 12 when the contact surface 26 of the housing 12 is not in contact with the skin S, the operator is prevented from erroneously pushing the pusher 128 and causing erroneous puncture by the puncture needle 72.

Note that the present invention is not limited to the above disclosure, and various configurations can be adopted without departing from the gist of the present invention.

## Claims

1. A liquid medicine administration device comprising:
a housing having a contact surface that has contact with skin of a living body;
a needle member that comprises a puncture needle and is movable relative to the housing, the puncture needle being capable of protruding from the contact surface toward the skin;
a syringe that is housed in the housing and comprises a cylindrical body, filled with a liquid medicine and connected to the puncture needle, a gasket movably housed in the cylindrical body, and a plunger having a distal end to which the gasket is coupled;
a drive unit that is housed in the housing and moves the plunger toward the distal end by being supplied with electric power;
a controller that is housed in the housing and controls the drive unit, the controller constituting a circuit with the puncture device comprising the needle member; and
a connection unit that connects the circuit at one position of a puncture position where the puncture needle has moved toward the skin and punctures the skin and a non-puncture position where the puncture needle does not puncture the skin, and disconnects the circuit at another position of the puncture position and the non-puncture position,
wherein the controller controls the drive unit to move the plunger toward the distal end when connection or disconnection of the circuit is detected as the puncture needle moves to the puncture position.

2. The liquid medicine administration device according to claim 1, wherein
the connection unit comprises a first circuit component and a second circuit component,
the first circuit component is made of a metal material and disposed on the needle member, and
the second circuit component is made of a metal material and disposed in the housing, the circuit is connected when the first circuit component and the second circuit component have contact with each other, and the circuit is disconnected when the first circuit component and the second circuit component are separated from each other.

3. The liquid medicine administration device according to claim 2, wherein
the circuit is connected when the first circuit component and the second circuit component have contact with each other at the non-puncture position, and
the circuit is disconnected when the first circuit component and the second circuit component are separated from each other at the puncture position.

4. The liquid medicine administration device according to claim 2, wherein
the circuit is disconnected when the first circuit component and the second circuit component are separated from each other at the non-puncture position, and
the circuit is connected when the first circuit component and the second circuit component have contact with each other at the puncture position.

5. The liquid medicine administration device according to any one of claims 2 to 4, comprising
an abutting member that has an abutting surface having contact with the skin and that is provided movably with respect to the housing, the abutting surface being capable of protruding from the contact surface,
wherein the second circuit component is disposed on the abutting member, and
the abutting member moves relative to the housing in such a manner as to maintain contact between the abutting surface and the skin when a relative position between the housing and a portion of the skin facing the abutting surface changes in a state where the housing has contact with the skin.

6. The liquid medicine administration device according to claim 5, wherein
the abutting member comprises a protruding portion that protrudes from the contact surface of the housing when the abutting surface of the abutting member does not have contact with the skin, the protruding portion has the abutting surface, and the abutting member is pushed by the skin to house the protruding portion in the housing when the contact surface of the housing has contact with the skin.

7. The liquid medicine administration device according to claim 5, comprising
a following biasing member that is provided in the housing and biases the abutting member toward the skin.

8. The liquid medicine administration device according to any one of claims 1 to 4, comprising
a puncture biasing member that is provided in the housing and biases the needle member toward the skin.

9. The liquid medicine administration device according to claim 5, comprising
a puncture mechanism that causes the needle member to protrude toward the skin from the abutting surface of the abutting member,
wherein the puncture mechanism comprises
a pusher that has a first inclined portion inclined with respect to a puncture direction of the puncture needle and is movable in the puncture direction with respect to the housing,
a puncture biasing member that is provided in the housing and biases the needle member toward the skin, and
a second inclined portion that is provided in the needle member, is inclined with respect to the puncture direction, and abuts on the first inclined portion,
the connection unit is disposed between the abutting member and the needle member,
the needle member rotates from a first position to a second position around an axis along the puncture direction, via the first inclined portion and the second inclined portion, as the pusher moves in the puncture direction, and
the needle member reaching the second position moves in the puncture direction by a biasing force of the puncture biasing member.

10. The liquid medicine administration device according to claim 9, wherein
the abutting member comprises a protruding portion protruding from the contact surface of the housing when the abutting surface of the abutting member does not have contact with the skin, and the protruding portion has the abutting surface,
the abutting member is pushed by the skin to house the protruding portion in the housing when the contact surface of the housing has contact with the skin, and
the pusher is housed in the housing when the abutting surface of the abutting member does not have contact with the skin, and protrudes from the housing when the contact surface of the housing has contact with the skin.

11. The liquid medicine administration device according to claim 8, wherein
the needle member comprises a needle hub that holds the puncture needle, and
the puncture biasing member is disposed between the abutting member and the needle hub.
